Europäisches Patentamt

European Patent Office

Office européen des brevets

(19)

(11) Veröffentlichungsnummer: **0 039 932**
**B1**

(12) EUROPÄISCHE PATENTSCHRIFT

(45) Veröffentlichungstag der Patentschrift:
25.09.85

(51) Int. Cl.⁴: **A 61 M 16/01**

(21) Anmeldenummer: 81103542.7

(22) Anmeldetag: 09.05.81

(54) Gasverhältnisregelvorrichtung für Narkosegeräte.

(30) Priorität: 14.05.80 US 149874

(43) Veröffentlichungstag der Anmeldung:
18.11.81 Patentblatt 81/46

(45) Bekanntmachung des Hinweises auf die Patenterteilung:
25.09.85 Patentblatt 85/39

(84) Benannte Vertragsstaaten:
DE FR GB SE

(56) Entgegenhaltungen:
AU - D - 5 852 373
DE - A - 1 931 354
DE - A - 2 225 683
DE - A - 2 706 857
DE - A - 2 740 067
GB - A - 2 001 249
US - A - 4 191 952

(73) Patentinhaber: Drägerwerk Aktiengesellschaft,
Moislinger Allee 53-55, D-2400 Lübeck 1 (DE)

(72) Erfinder: Schreiber, Peter Jürgen, Ing., Box 74, R.D. 1,
Zionsville Pennsylvania 18092 (US)

## Beschreibung

Die Erfindung betrifft eine Gasverhältnisregelvorrichtung zur Verwendung in Narkosegeräten, entsprechend dem Gattungsbegriff des Anspruches 1.

Handelsübliche Narkosegeräte besitzen Einstellventile für den Durchfluss von Sauerstoff und Narkosegas(en), z.B. Lachgas, in eine gemeinsame Sammelleitung und von dort zum Atemkreislauf des Patienten. Die meisten Geräte besitzen auch Fühler und Anzeigeinstrumente, z.B. Durchflussmesser, die den Durchfluss durch die Einstellventile anzeigen, sowie Anzeigeinstrumente für andere Messgrössen, z.B. den Gasdruck.

Es liegt in der Verantwortlichkeit des Benutzers des Narkosegerätes, eine Mindestversorgung mit Sauerstoff in dem abgegebenen Gasfluss zu sichern. Ungeachtet dieser Verantwortlichkeit ereigneten sich in den letzten Jahren verschiedene Unfälle, weil der Sauerstoffgehalt die untere Sicherheitsgrenze unterschritt. Manche derartigen Unfälle beruhten auf dem Ausbleiben der Sauerstoffzufuhr, versehentlichem Schliessen des Sauerstoff-Einstellventils oder einer Fehleinschätzung in der Einstellung der Durchflüsse.

Verschiedene Sicherheitseinrichtungen sind bekannt und handelsüblich, die auf den Druck in der Sauerstoffzuleitung ansprechen. Solche Einrichtungen zeigen ein Absinken oder völliges Ausbleiben des Sauerstoffversorgungsdruckes an. Solche Einrichtungen können auch bei einem teilweisen oder völligen Ausbleiben des Sauerstoffversorgungsdruckes alle anderen Gasflüsse unterbrechen oder einschränken. Jedoch haben die bekannten Einrichtungen, die auf den Sauerstoffdruck ansprechen, den grossen Nachteil, dass bei geschlossenem Sauerstoffeinstellventil, wenn also dem Patienten kein Sauerstoff zugeführt wird, der Sauerstoffdruck in der Zuleitung weiterhin vorhanden ist und die Warneinrichtung keinen Alarm auslöst, obwohl kein Sauerstoff fliesst.

In der US-A-4 191 952 ist eine Warneinrichtung zur Verwendung in Narkosegeräten beschrieben, die das Gasverhältnis überwacht und die im Oberbegriff des Anspruchs 1 genannten Merkmale besitzt. Dabei werden Sauerstoff und Narkosegas, an unabhängig einstellbaren Einstellorganen geregelt, über Leitungen in eine Sammelleitung zugeführt. Die Warneinrichtung enthält ein 1. druckbetätigtes Mittel für den Sauerstoffdruck in der 1. Leitung mit einem Ausgangselement, dessen Stellung vom Sauerstoffdruck abhängt, ein 2. druckbetätigtes Mittel für den Narkosegasdruck in der 2. Leitung mit einem Ausgangselement, dessen Stellung vom Narkosegasdruck abhängt, und eine Warnvorrichtung. Die Warnvorrichtung besitzt ein verschiebbares Teil. Die beiden Ausgangselemente sind mit dem verschiebbaren Teil verbunden und wirken gegenläufig auf dieses zur Verschiebung in einer 1. Richtung und in einer dazu entgegengesetzten 2. Richtung ein. Die Warnvorrichtung enthält einen Schalter, der ein Alarmsignal abgibt, sobald das verschiebbare Teil in der 2. Richtung bis zu einer festgelegten Stellung verschoben ist. Es handelt sich hier eindeutig nur um eine Warneinrichtung mit den beiden Zuständen: entweder keine Warnung; oder Warnung, d.h. die $O_2$-Konzentration liegt unterhalb eines vorgegebenen Grenzwertes. Es fehlt das für eine Regeleinrichtung notwendige Stellglied zur kontinuierlichen Regelung des Gasflusses durch die 2. Leitung in Abhängigkeit von dem herrschenden Differenzdruck zwischen den beiden druckbetätigten Mitteln.

Die Patentanmeldung GB-A-2 001 249 beschreibt eine Warn- und Schutzeinrichtung in einem Narkosesystem. Es besitzt Einrichtungen, mit denen eine Patientenleitung mit 2 Gasen, die im Narkoseprozess benutzt werden, verbunden wird. Dieses bedeutet einmal die Verbindung über einen 1. Gasanschluss mit einem druckempfindlichen Element, das wiederum mit einem Ventil in dem 2. Gasanschluss verbunden ist. Die Anordnung ist derart, dass bei einem Druckabfall in der 1. Gasleitung unter einen vorbestimmten Wert das Ventil in der 2. Gasleitung geschlossen wird, so dass hier kein Gas mehr fliessen kann.

Ein Reservegasbehälter, der mit dem 1. Gas gefüllt ist und unter dem Normaldruck des 1. Gases steht, entleert sich bei Druckabfall in die 1. Leitung hinein und versorgt diese über eine kurze Zeit hinweg mit Gas. Gleichzeitig wird ein Alarm ausgelöst.

Nachteilig ist, dass nur in der 1. Gasleitung, z.B. dem Sauerstoffanschluss, ein druckempfindliches Element vorhanden ist und daher nur Druckänderungen in der Sauerstoffleitung das Ventil in der 2. Gasleitung, für das Narkosegas, betätigen und dort den Druckfluss ändern. Eine Druckänderung im Narkosegas wirkt sich nicht aus. In der US-A-4 015 617 ist ein Narkosegerät beschrieben, das eine Mischung von Sauerstoff und Lachgas in den Atemkreislauf eines Patienten liefert. Das Gerät enthält einen einstellbaren Regler für den Sauerstoffdurchfluss in den Atemkreislauf und einen Druckminderer, der den Lachgasdurchfluss in Abhängigkeit von dem überwachten Sauerstoffdruck regelt. Durch Veränderung des Reglers für den Sauerstoffdurchfluss wird automatisch auch der Lachgasdurchfluss verändert, um ein festgesetztes Durchflussverhältnis beizubehalten. Ein Nadelventil in der Lachgasleitung hinter dem Druckminderer ermöglicht eine Drosselung des Lachgasdurchflusses und damit eine Veränderung des Mischungsverhältnisses.

Nachteilig daran ist die begrenzte Verwendbarkeit, weil der Lachgas- und Sauerstoffdurchfluss über die Einstellung miteinander verknüpft sind. Denn bei einer Verringerung des Sauerstoffdurchflusses erfolgt automatisch eine entsprechende Verringerung des Lachgasdurchflusses. Erst durch eine zusätzliche Betätigung des Nadelventils kann über die Änderung des Mischungsverhältnisses der ursprüngliche Lachgasdurchfluss wiederhergestellt werden.

Somit besteht ein Bedarf für eine Gasverhältnisregelvorrichtung für Narkosegeräte, die automatisch das Verhältnis von Sauerstoff zu Narkosegas, die dem Atemkreislauf des Patienten zugeführt werden, regelt und dabei eine unabhängige Einstellung erlaubt, solange ein Grenzwert des Sauerstoffdurchflusses eingehalten ist.

Aufgabe der Erfindung ist daher eine Gasverhältnisregelvorrichtung für Narkosegeräte, die bei einfacher Bauform die unabhängige Einstellung von Sauerstoff und Narkosegas erlaubt, solange der Sauerstoffdurchfluss über einem vorgegebenen Grenzwert liegt.

Die Lösung der Aufgabe erfolgt bei einer Vorrichtung der im Oberbegriff des Anspruchs 1 genannten Art gemäss dem Kennzeichen des Anspruches 1. Vorteilhafte Weiterbildungen ergeben sich aus den Ansprüchen 2 bis 6.

Die Ausgestaltungen und die erzielten Vorteile werden anhand des Ausführungsbeispiels deutlich, das in der Zeichnung dargestellt und im folgenden beschrieben ist. Es zeigen

Fig. 1 einen Teil eines Narkosegerätes mit der erfindungsgemässen Gasverhältnisregelvorrichtung in schematischer Darstellung,

Fig. 2 einen Durchflussverhältnisregler im Schnitt.

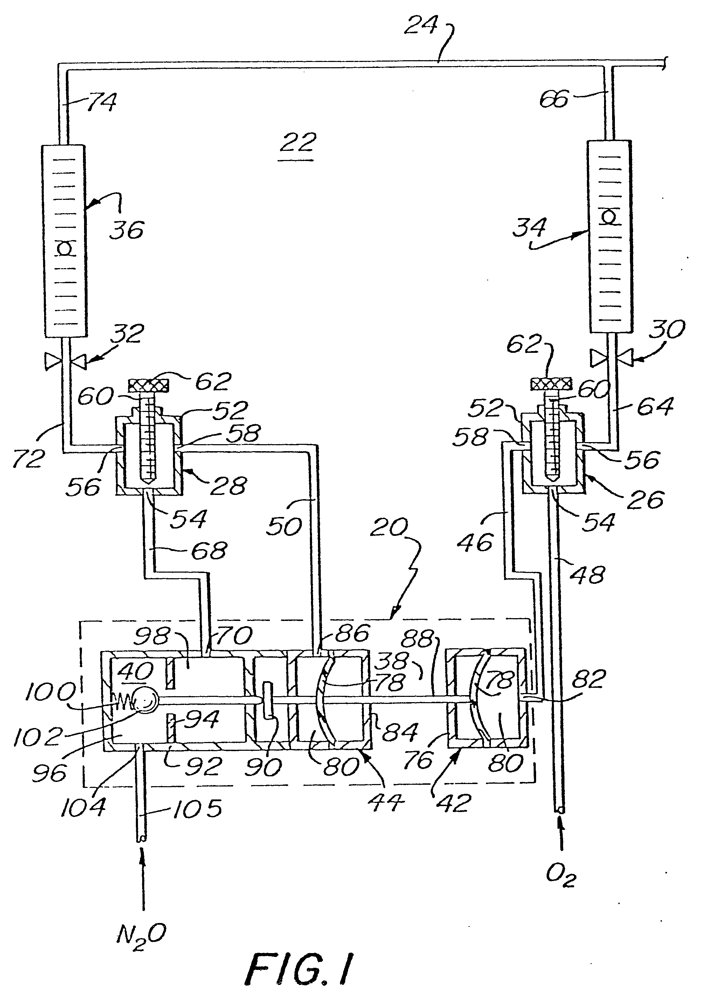

In Fig. 1 ist ein Durchflussverhältnisregler 20 in einem Narkosegerät 22 gezeigt (von dem nur der interessierende Teil gezeigt und nachfolgend beschrieben ist). Das Narkosegerät 22 ist von üblicher Bauart und führt dem Patienten ein Gemisch von Sauerstoff und Narkosegas(en) durch eine Sammelleitung 24 zur Atmung zu. Hierzu enthält das Narkosegerät 22 eine Sauerstoff- und eine Narkosegasflasche (beide nicht dargestellt). Sauerstoff und Narkosegas, wie z.B. Lachgas, strömen über jeweilige Einstellventile 26, 28, Drosseln 30, 32 und Durchflussmesser 34, 36 in die gemeinsame Sammelleitung 24, wo sich die Gase zur Zufuhr zu dem (nicht dargestellten) Atemkreislauf des Patienten mischen. Die Einstellventile sind von Hand einstellbar, um den Gasdurchfluss zu bestimmen. Jede Drossel besteht aus einer verringerten Öffnung als lineare Drossel. Die Durchflussmesser 34, 36 zeigen den Gasdurchfluss in die Sammelleitung 24.

Der Durchflussverhältnisregler 20 enthält einen Differenz-Druckaufnehmer 38 und einen Durchflussregler 40. Der später ausführlich beschriebene Differenz-Druckaufnehmer 38 überwacht den Sauerstoffanteil in der Sammelleitung 24. Dies erfolgt durch Vergleich des Sauerstoffdruckes aus dem Durchfluss des Sauerstoffes durch die Drossel 30 mit dem Lachgasdruck aus dem Lachgasdurchfluss durch die Drossel 32. Der Durchflussregler 40 bildet ein Ventil, um den Lachgasdurchfluss zum Lachgas-Einstellventil 28 durch den Differenz-Druckaufnehmer 38 zu regeln.

Der Differenz-Druckaufnehmer 38 enthält einen Sauerstoff-Druckaufnehmer 42 und einen Narkosegas-Druckaufnehmer 44. Der Sauerstoff-Druckaufnehmer 42 enthält eine Membrangruppe, die über eine Steuerleitung 46 von dem Sauerstoff-Einstellventil 26 stromaufwärts der Drossel 30 unter Druck gesetzt wird. Der Narkosegas-Druckaufnehmer 44 enthält ebenfalls eine Membrangruppe, die über eine Steuerleitung 50 vom Lachgas-Einstellventil 28 stromaufwärts der Drossel 32 unter Druck gesetzt wird.

Die Einstellventile 26, 28 sind von üblicher Bauart und enthalten jeweils ein Gehäuse 52 mit einem Gaseinlass 54, einem Gasauslass 56, einem Steueranschluss 58 und einem einstellbaren Nadelventil 60. Ein Handrad 62 am Nadelventil 60 dient der Einstellung des Sauerstoff- bzw. Lachgasdurchflusses. Eine Leitung 48 zum Gaseinlass 54 des Sauerstoff-Einstellventils 26 führt Sauerstoff von der Sauerstoffflasche zu, der über den Gasauslass 56 abgegeben wird. Die Steuerleitung 46 verbindet den Steueranschluss 58 mit dem Sauerstoff-Druckaufnehmer 42 des Differenz-Druckaufnehmers 38. Eine Abgangsleitung 64 verbindet den Gasauslass 56 mit der Drossel 30.

Die beiden Durchflussmesser 34, 36 sind von üblicher Bauart und enthalten ein konisches Glasrohr mit in aufwärtiger Richtung erweitertem Innendurchmesser. Das Glasrohr enthält als Durchflussanzeiger einen frei beweglichen Schwimmer. Das Glasrohr ist mit einer Teilung in Volumen/Zeiteinheit versehen.

Wie aus Fig. 1 ersichtlich, ist das obere Ende des Durchflussmessers 34 mit einer Zweigleitung 66 verbunden, die die Sauerstoffzuleitung zu der Sammelleitung 24 bildet. Der Gaseinlass 54 des Lachgas-Einstellventils 28 ist an eine Verbindungsleitung 68 angeschlossen, die mit dem Auslass 70 des Durchflussreglers 40 verbunden ist und von dort mit Lachgas aus der Gasflasche versorgt wird. Die Steuerleitung 50 verbindet den Steueranschluss 58 des Lachgas-Einstellventils 28 mit dem Narkosegas-Druckaufnehmer 44 des Differenz-Druckaufnehmers 38. Eine Abgangsleitung 72 verbindet den Gasauslass 56 des Lachgas-Einstellventils 28 mit der Drossel 32. Der Durchflussmesser 36 ist zwischen die Drossel 32 und eine Zweigleitung 74 eingeschaltet, die als zweite Zuleitung in die Sammelleitung 24 dient.

Der Durchflussverhältnisregler 20 regelt und überwacht den Sauerstoffanteil in dem Sauerstoff-Lachgasgemisch. Die Sauerstoffkonzentration wird durch Vergleich der Durchflüsse von Sauerstoff und Lachgas in die Sammelleitung 24 überwacht. Dies erfolgt durch Vergleich des Sauerstoffdruckes und des Lachgasdruckes, die von den Durchflüssen der Gase durch die jeweiligen Drosseln 30, 32 herrühren. Bekanntlich sind die Drücke in den Gehäusen der Einstellventile 26, 28 jeweils eine Funktion des Widerstandes der Drossel und des Gasdurchflusses durch die Drossel. Generell ist diese Beziehung nicht linear, aber ein Anstieg des Durchflusses bewirkt stets einen Anstieg des Druckes. Folglich steht das Verhältnis des Sauerstoffdruckes zum Lachgasdruck in Beziehung zur Konzentration in der Sammelleitung 24.

Das Druckverhältnis wird überwacht durch den Differenz-Druckaufnehmer 38. Wie erwähnt, besteht der Differenz-Druckaufnehmer 38 aus den beiden Druckaufnehmern 42, 44. Die Druckaufnehmer besitzen jeder ein besonderes Gehäuse und sind getrennt voneinander, um eine Trennung von Sauerstoff und Lachgas zu sichern. Deshalb enthält der Sauerstoff-Druckaufnehmer 42 ein Gehäuse 76 mit einer Membrangruppe 78, die darin eine Druckkammer 80 abteilt. Das Gehäuse 76 enthält einen Steueranschluss 82, der die Steuerleitung 46 mit der Druckkammer 80 verbindet. Der Narkosegas-Druckaufnehmer 44 ist von ähnlicher Konstruktion und besitzt eine Membrangruppe 78, die innerhalb eines Gehäuses 84 eine Druckkammer 80 abteilt. Das Gehäuse 84 enthält einen Steueranschluss 86, der die Steuerleitung 50 mit der Druckkammer 80 des Narkosegas-Druckaufnehmers 44 verbindet.

Die Membrangruppen 78 der Druckaufnehmer 42, 44 sind durch ein Verbindungsstück 88 miteinander verbunden. Das Verbindungsstück 88 besitzt ein Druckstück 90, das als Ausgangselement des Differenz-Druckaufnehmers 38 dient und auf den Durchflussregler 40 einwirkt. Das Druckstück 90 kann in Längsrichtung, also von rechts nach links und umgekehrt, entsprechend dem herrschenden Differenzdruck bewegt werden, wie er durch den Differenz-Druckaufnehmer 38 überwacht wird. Die Stellung des Druckstückes 90 ist damit ein Mass für den herrschenden Differenzdruck und die zugehörigen Gasdurchflüsse. Die Stellung des Druckstückes 90 bestimmt die Grösse der Öffnung des Durchflussreglers 40 von voller Öffnung über teilweise Öffnung bis zu vollem Verschluss und bestimmt damit den Durchfluss des Narkosegases durch den Durchflussregler.

Der Durchflussregler 40 enthält in einem dichten Gehäuse 92 einen Ventilsitz 94, der das Gehäuse in eine Einlasskammer 96 und eine Auslasskammer 98 teilt. Eine Schliessfeder 100 und ein beweglicher Ventilkörper 102 sind in der Einlasskammer 96 angeordnet. Das Druckstück 90 regelt die Stellung des Ventilkörpers 102 bezüglich des Ventilsitzes 94 gegen die Kraft der Schliessfeder 100. Das Gehäuse 92 enthält einen Einlass 104 an der Einlasskammer 96. Der vorerwähnte Auslass 70 ist mit der Auslasskammer 98 verbunden. Eine Leitung 105 ist mit dem Einlass 104 verbunden und leitet Lachgas von der Gasflasche über den Durchflussregler 40 zu seinem Auslass 70.

Wie erwähnt, verbindet das Verbindungsstück 88 die Membrangruppen der Druckaufnehmer 42, 44 miteinander. Diese Baugruppen sind einander entgegengesetzt angeordnet, so dass die Bewegung der Membrangruppe 78 des Sauerstoff-Druckaufnehmers 42 unter dem Sauerstoffdruck der Bewegung der Membrangruppe 78 des Narkosegas-Druckaufnehmers 44 unter dem Lachgasdruck entgegengesetzt ist. Da der Sauerstoffdruck in der Steuerleitung 46 und der Lachgasdruck in der Steuerleitung 50 nicht nur von den jeweiligen Sauerstoff- und Lachgasdurchflüssen durch die betreffenden Einstell-Ventile 26, 28 abhängen, sondern auch von den Widerstandswerten der jeweiligen Drosseln 30, 32, ist der Widerstand der Drosseln so gewählt, dass das Verbindungsstück 88 die in Fig. 1 dargestellte 1. Stellung einnimmt, wenn das Verhältnis des Sauerstoffdurchflusses zum Lachgasdurchfluss (und folglich der Sauerstoffkonzentration im Atemgas in der Sammelleitung) einen festgesetzten Grenzwert, z.B. 25%, überschreitet. Das Verbindungsstück 88 bewegt sich in entgegengesetzte Richtung, d.h. in eine 2. oder geschlossene Stellung, wenn das Verhältnis des Sauerstoffdurchflusses unter diesen festgesetzten Wert sinkt. In der 1. Stellung des Verbindungsstückes hebt das Druckstück 90 den Ventilkörper 102 völlig vom Ventilsitz 94, so dass der Durchflussregler 40 voll geöffnet ist. Wenn das Verbindungsstück in der 2. Stellung ist, bewirkt die Kraft der Schliessfeder 100 ein Aufliegen des Ventilkörpers 102 auf dem Ventilsitz 94 und dadurch ein Schliessen des Durchflussreglers 40. Wenn das Verbindungsstück sich in einer beliebigen Zwischenstellung zwischen der 1. und 2. Stellung befindet, ist der Durchflussregler teilweise geöffnet, wobei die Grösse der Öffnung abhängig von der Stellung des Verbindungsstückes ist.

Die Funktion des Verbindungsstückes 88 bei der Betätigung des Durchflussreglers 40 ist wie folgt: Falls das Verhältnis des Sauerstoffdurchflusses zum Lachgasdurchfluss oberhalb des festgesetzten Grenzwertes von z.B. 25% ist, überwiegt die von dem Druck auf die Membran des Sauerstoff-Druckaufnehmers 42 ausgeübte Kraft die Kraft, die von dem Druck auf die Membran des Narkosegas-Druckaufnehmers ausgeübt wird. Dann bewegt sich das Verbindungsstück 88 nach links in die in Fig. 1 dargestellte 1. Stellung, worauf das Druckstück 90 den Ventilkörper 102 im Durchflussregler 40 gegen die Kraft der Schliessfeder 100 voll von dem Ventilsitz 94 abhebt und dadurch Lachgas vom Auslass 70 des Durchflussreglers zum Lachgaseinstellventil 28 fliessen lässt. In diesem Zustand ist der Durchflussregler 40 voll geöffnet und gestattet eine ungehinderte Steuerung des Lachgasdurchflusses innerhalb der Sicherheitsgrenzen, die durch die Drosseln 30, 32 vorgegeben sind. Solange der Durchflussregler 40 voll geöffnet ist, kann entweder der Sauerstoff- oder der Narkosegasdurchfluss unabhängig an dem jeweiligen Einstellventil 26, 28 eingestellt werden.

Falls der Lachgasdurchfluss über die Sicherheitsgrenzen ansteigt oder der Sauerstoffdurchfluss unter die Sicherheitsgrenzen sinkt, überwiegt die von dem Druck auf die Membrangruppe 78 des Narkosegas-Druckaufnehmers 44 ausgeübte Kraft diejenige Kraft, die von dem Druck auf die Membrangruppe 78 des Sauerstoff-Druckaufnehmers 42 ausgeübt wird. Daraufhin bewegt sich das Druckstück 90 nach rechts in eine Zwischenstellung, so dass der Ventilkörper sich dem Ventilsitz nähert und der Durchflussregler 40 teilweise geöffnet ist. In diesem Fall wirken der Differenz-Druckaufnehmer 38 und der Durchflussregler 40 als ein hilfsdruckgesteuerter (slave pressure) Regler, wobei die entgegengesetzten Membrankräfte die Grösse der Ventilöffnung so regeln, dass das Verhältnis des Sauerstoffdurchflusses zum Lachgasdurchfluss auf dem Grenzwert gehalten wird, ungeachtet der durch die Einstellung der jeweiligen Einstellventile 26, 28 vorgegebenen Durchflussmenge von Sauerstoff und Lachgas.

Falls der Sauerstoffdurchfluss unterbrochen wird, wie es geschehen kann, wenn das Sauerstoff-Einstellventil 26 zufällig geschlossen wird, überwiegt die von der Membran des Narkosegas-Druckaufnehmers 44 und der Schliessfeder 100 ausgeübte Kraft die Kraft der Membran des Sauerstoff-Druckaufnehmers 42, und das Verbindungsstück bewegt sich in die 2. Stellung, in der der Ventilkörper 102 auf dem Ventilsitz ruht und der Durchflussregler 40 geschlossen ist.

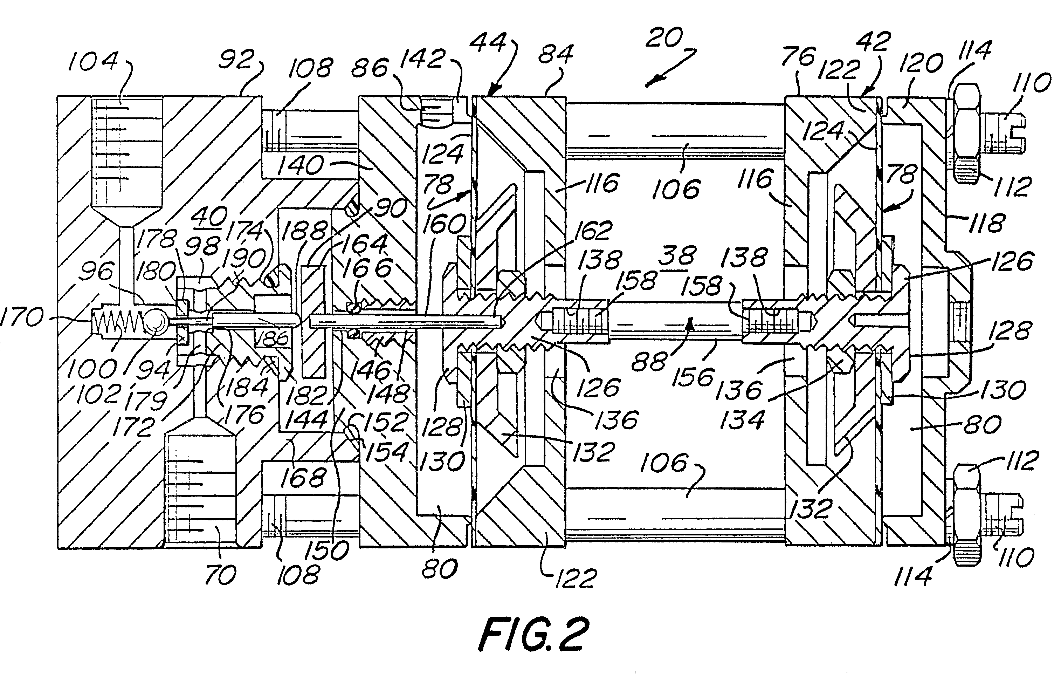

Anhand der Fig. 2 werden die Einzelheiten des Differenz-Druckaufnehmers 38 und des Durchflussreglers 40 beschrieben. Darin enthalten der Sauerstoff-Druckaufnehmer 42 ein Gehäuse 76 und der Narkosegas-Druckaufnehmer 44 ein Gehäuse 84. Der Durchflussregler 40 enthält ein Gehäuse 92. Die Gehäuse 76, 84 und 92 sind miteinander durch mehrere in Längsrichtung angeordnete Bolzen 106 verbunden. Das freie Ende jedes Bolzens besitzt ein Gewinde 108. Jeder Bolzen erstreckt sich durch fluchtende Öffnungen in den Gehäusen 76, 84 und ist mit dem Gewinde 108 in einem Gewinde des Gehäuses 92 des Durchflussreglers 40 verankert. Das entgegengesetzte Ende 110 jedes Bolzens ist als Schlitzschraube ausgeführt und trägt eine Sechskantmutter 112 und eine Sicherungsscheibe 114, um die Gehäuse zusammenzuspannen.

Das Gehäuse 76 enthält zwei hohle Bauteile 116, 118. Bauteil 118 enthält einen ringförmigen Flansch 120 und Bauteil 116 einen ringförmigen Flansch 122. Die Membrangruppe 78 in dem Sauerstoff-Druckaufnehmer 42 enthält eine Membran 124 aus elastischem Material, deren Umfang zwischen den Flanschen 120, 122 des Gehäuses 76 dicht eingespannt ist. Die Membran 124 und die Innenwand des Bauteils 118 bilden die Druckkammer 80 des Sauerstoff-Druckaufnehmers 42. Die Membran 124 besitzt eine zentrale Öffnung, durch die sich ein rohrförmiges Verbindungselement 126 erstreckt. Das Verbindungselement hat einen grösseren sechskantförmigen Kopf 128. Eine Unterlegscheibe 130 liegt zwischen dem Kopf 128 und der Umgebung der zentralen Öffnung in der Membran 124. Eine Scheibe 132 mit abgewinkeltem Umfang ist mit einer zentralen Öffnung auf dem Verbindungselement 126 an der der Unterlegscheibe gegenüberliegenden Membranseite montiert. Die Aussenseite des Verbindungselements 126 ist teilweise mit Gewinde versehen und darauf eine Sechskantmutter 134 befestigt. Das Anziehen der Sechskantmutter spannt den um die zentrale Öffnung gelegenen Membranabschnitt damit zwischen der Scheibe 132 und der Unterlegscheibe 130 ein, um die Druckkammer 80 abzudichten. Das freie Ende des Verbindungselements 126 bildet einen dünneren Ansatz und durchdringt das Gehäusebauteil 116 in einer zentralen Öffnung 136. Der dünnere Ansatz des Verbindungselements 126 enthält eine Längsbohrung 138 mit Gewinde, die bei der Verbindung der Membrangruppen miteinander durch das Verbindungsstück 88 als Befestigung dient.

Das Gehäuse 84 des Narkosegas-Druckaufnehmers 44 ist, mit Ausnahme eines Abschnitts, von ähnlicher Bauweise wie das Gehäuse 76 des Sauerstoff-Druckaufnehmers 42. Deshalb sind alle Einzelteile des Gehäuses 84, die mit den Einzelteilen des Sauerstoff-Druckaufnehmers 42 übereinstimmen mit denselben Bezugszahlen gekennzeichnet, und ihre Beschreibung bedarf keiner Wiederholung.

Das Gehäuse 84 enthält das bereits genannte Bauteil 116 und ein Bauteil 140. Das Bauteil 140 enthält einen Flansch 142, ähnlich dem Flansch 120 des Sauerstoff-Druckaufnehmers 42. Ebenso ist die Membrangruppe 78 des Narkosegas-Druckaufnehmers 44 dicht zwischen den gegenüberliegenden Flanschen 142, 122 eingespannt. Die Druckkammer 80 des Narkosegas-Druckaufnehmers 44 wird zwischen der inneren Oberfläche des Bauteils 140 und der Membran 124 gebildet. Der Steueranschluss 86 erstreckt sich durch den Flansch 142 in die Druckkammer 80. Das Bauteil 140 enthält eine zentrale Öffnung 144, in die ein Nippel 146 mit Gewinde eingesetzt ist. Der Nippel enthält eine glatte zentrale Öffnung 148, die von einem Abschnitt des Druckstückes 90 durchsetzt ist. Das Bauteil 140 enthält einen zentralen Ansatz 150 mit einem ringförmigen Einstich 152 an seinem Umfang. Ein O-Ring 154 liegt in dem Einstich und bildet eine Abdichtung gegenüber einem anliegenden Abschnitt des Durchflussreglers 40.

Wie erwähnt, verbindet das Verbindungsstück 88 den Sauerstoff-Druckaufnehmer 42 mit dem Narkosegas-Druckaufnehmer 44. Hierzu besitzt das Verbindungsstück 88 einen gestreckten Schaft 156 mit einem Paar Gewinden 158 an den Enden. Ein Gewinde 158 ist in die Längsbohrung 138 des Verbindungselements eingeschraubt, das zu der Membrangruppe im Narkosegas-Druckaufnehmer 44 gehört, während das gegenüberliegende Gewinde des Schaftes 156 in das Verbindungselement der Membrangruppe des Sauerstoff-Druckaufnehmers 42 eingeschraubt ist. Auf diese Weise sind die Membrangruppen miteinander sicher verbunden und können sich nicht unabhängig voneinander bewegen.

Das Druckstück 90 enthält einen gestreckten Druckbolzen 160, dessen eines Ende in einer Längsbohrung 162 in dem Verbindungselement 126 des Narkosegas-Druckaufnehmers 44 gehalten ist. Das entgegengesetzte Ende des Druckbolzens 160 erstreckt sich durch den Nippel 146 und das Bauteil 140 bis in die zentrale Öffnung einer Druckscheibe 164. Ein O-Ring 166 ist in einer Nut im Bauteil 140 nahe dem herausragenden Ende des Druckbolzens 160 angeordnet, um das Innere der Druckkammer 80 abzudichten.

Der Durchflussregler 40 enthält ein Gehäuse 92, an dessen innerer Seite ein rohrförmiger Vorsprung 168 angebracht ist, der den Ansatz 150 des Narkosegas-Druckaufnehmers 44 in seinem Inneren aufnimmt. Mit dem zwischen dem Vorsprung 168 und dem diesem anliegenden Abschnitt des Bauteils 140 eingefügten O-Ring wird der Raum mit der Druckscheibe gegen die Umgebung abgedichtet.

Die Einlasskammer 96 des Durchflussreglers 40 ist als rohrförmige Öffnung ausgebildet. Die schraubenförmige Schliessfeder 100 und der Ventilkörper 102 sind in dieser Öffnung angeordnet. Der Durchmesser der Einlasskammer 96 ist etwas grösser als der Durchmesser des Ventilkörpers 102, damit sich dieser darin in Länsrichtung bewegen kann. Die Schliessfeder 100 liegt zwischen dem Ventilkörper und der Rückwand 170 der Einlasskammer 96. Der Ventilsitz 94 liegt der Vorderseite der Einlasskammer 96 an und ist durch einen Gewindeeinsatz 172 festgehalten. Der Gewindeeinsatz 172 ist in eine Gewindeöffnung 174 im Gehäuse 92, die sich an die Einlasskammer 96 anschliesst, eingeschraubt. Der Gewindeeinsatz 172 enthält einen zentralen Durchgang 176, der an einer Seite in einer Erweiterung 178 endet, in welcher der Ventilsitz 94 liegt. Der Gewindeeinsatz 172 hat nahe dem Ventilsitz einen Ansatz von geringerem Durchmesser, um die Auslasskammer 98 zu bilden. Mehrere radiale Öffnungen 179 bilden eine Verbindung zwischen der ringförmigen Auslasskammer 98 und dem zentralen Durchgang 176 in dem Gewindeeinsatz 172. Der Ventilsitz 94 enthält eine zentrale Öffnung 180, die mit der Auslasskammer 98 verbunden ist. Der Auslass 70 des Durchflussreglers 40 erstreckt sich durch das Gehäuse 92 bis zur Auslasskammer 98. Der Gewindeeinsatz 172 besitzt einen überstehenden Flansch 182. Ein O-Ring 184 bildet die Abdichtung zwischen dem Flansch und dem Gehäuse 92. Der Einlass 104 des Durchflussreglers 40 steht in Verbindung mit der Einlasskammer 96.

Zum Druckstück 90 gehört ausserdem ein Druckstift 186 in dem Durchgang 176 des Gewindeeinsatzes 172. Der Druckstift 186 ist ein längliches Bauteil mit einem gerundeten Ende 188, das der Druckscheibe 164, und einem Ansatz 190 von geringerem Durchmesser, der dem Ventilkörper 102 des Durchflussreglers 40 anliegt. Der Druckstift 186 kann von der Druckscheibe 164 in dem Durchgang 176 hin und her bewegt werden.

Die Funktion des Differenz-Druckaufnehmers 38 und des Durchflussreglers 40 ist wie folgt: Wenn das über die Steuerleitungen 46, 50 überwachte Druckverhältnis den gewünschten Grenzwert, z.B. 25% Sauerstoff, überschreitet, übersteigt der Druck in der Druckkammer 80 des Sauerstoff-Druckaufnehmers 42 den Druck in der Druckkammer 80 des Narkosegas-Druckaufnehmers 44. Dann bewegt sich die Membran 124 des Sauerstoff-Druckaufnehmers 42 auswärts und die Membran des Narkosegas-Druckaufnehmers 44 einwärts. Da beide Membrangruppen miteinander durch den Schaft 156 des Verbindungsstückes 88 verbunden sind, bewegt sich das Verbindungsstück 88 nach links in die in Fig. 1 gezeigte Stellung. Die Linksbewegung des Verbindungsstückes 88 bewegt den Druckbolzen 160 nach links. Die dadurch nach links bewegte Druckscheibe 164 berührt das gerundete Ende 188 des Druckstiftes 186 und bewegt den Druckstift 186 nach links, wobei sein Ansatz 190 den Ventilkörper 102 berührt und diesen gegen die Kraft der Schliessfeder 100 von seinem Sitz abhebt. Dadurch kann Lachgas vom Einlass 104 des Durchflussreglers 40 durch die Einlasskammer 96, über die zentrale Öffnung 180 im Ventilsitz, die Auslasskammer 98 und den Auslass 70 zum Lachgas-Einstellventil 28 fliessen.

Wenn eines der Einstellventile 26, 28 so eingestellt ist, dass das Verhältnis von Sauerstoffdurchfluss zu Lachgasdurchfluss unterhalb des Grenzwertes liegt, bilden sich in den Druckkammern 80 des Sauerstoff-Druckaufnehmers 42 und Narkosegas-Druckaufnehmers 44 die Drücke derart aus, dass sich das Verbindungsstück 88 nach rechts bewegt und sich der Ventilkörper 102 dem Ventilsitz nähert, so dass der Durchflussregler teilweise geöffnet ist. Dieser Vorgang bewirkt eine Regelung des Lachgasdurchflusses durch den Durchflussregler 40, so dass das Verhältnis des Sauerstoff- und Lachgasdurchflusses in die Sammelleitung auf dem gewünschten Grenzwert gehalten wird.

Falls der Sauerstoffdurchfluss unterbrochen wird, bewegt der Differenz-Druckaufnehmer den Ventilkörper 102 auf den Ventilsitz 94, wodurch der Zufluss des Lachgases zum Lachgas-Einstellventil 28 unterbrochen wird. In diesem Fall wird das Bedienungspersonal auf die Gefahrensituation durch eine nicht dargestellte Alarmeinrichtung hingewiesen.

Es sei darauf hingewiesen, dass anstelle der geschilderten mechanischen Hilfsmittel zur Regelung des Lachgasdurchflusses in Abhängigkeit von den überwachten Sauerstoff- und Lachgasdurchflüssen ebenso auch elektrische Hilfsmittel, z.B. Halbleiter, angewendet werden können, um Signale entsprechend dem Differenzdruck zu erzeugen und einen Regler für Lachgas entsprechend diesem Differenzdruck zu öffnen oder zu schliessen.

Ebenso kann die Erfindung zum Gebrauch mit mehr als einem Narkosegas abgewandelt werden. Die erfindungsgemässe Gasverhältnisregelvorrichtung ist von einfacher Bauweise und bietet einen weiten Anwendungsbereich, da sie eine automatische Überwachung und Regelung des Verhältnisses von Sauerstoff und Narkosegas, die dem Atemkreislauf des Patienten zugeführt werden, bewirkt, ohne dafür eine unabhängige Einstellung sowohl des Sauerstoffs wie des Narkosegases zu opfern.

**Patentansprüche**

1. Gasverhältnisregelvorrichtung zur Verwendung in Narkosegeräten mit einer 1. Leitung (48, 64) zur Einspeisung von Sauerstoff und einer 2. Leitung (105, 68, 72) zur Einspeisung von Narkosegas in eine Sammelleitung (24), mit je einem unabhängig einstellbaren Einstellorgan (26, 28) für den Gasfluss in beiden Leitungen, mit einem Sauerstoff-Druckaufnehmer (42) an der 1. Leitung hinter dem Einstellorgan (26), der ein 1. Signal entsprechend dem Sauerstoffdruck abgibt, mit einem Narkosegas-Druckaufnehmer (44) an der 2. Leitung hinter dem Einstellorgan (628), der ein 2. Signal entsprechend dem Narkosegasdruck abgibt, und mit einer auf die von den beiden Druckaufnehmern (42, 44) überwachte Druckdifferenz ansprechenden Verstelleinrichtung (160, 90, 186, 190), dadurch gekennzeichnet, dass sie einen mit der 2. Leitung (105, 68) verbundenen Durchflussregler (40) zur Regelung des Gasdurchflusses durch die 2. Leitung enthält, auf den die Verstelleinrichtung (160, 90, 186, 190) einwirkt.

2. Gasverhältnisregelvorrichtung nach Anspruch 1, dadurch gekennzeichnet, dass der Sauerstoff-Druckaufnehmer (42) ein Ausgangselement, dessen Stellung vom Sauerstoffdruck hinter dem Einstellorgan (26) abhängt und das 1. Signal darstellt, der Narkosegas-Druckaufnehmer (44) ein Ausgangselement, dessen Stellung vom Narkosegasdruck hinter dem Einstellorgan (28) abhängt und das 2. Signal darstellt, und die Verstelleinrichtung ein verschiebbares Teil (90) besitzt, das mit den beiden Ausgangselementen und dem Durchflussregler (40) verbunden ist, wobei das Ausgangselement des Sauerstoff-Druckaufnehmers (42) bei steigendem Sauerstoffdruck im öffnenden und das Ausgangselement des Narkosegas-Druckaufnehmers (44) bei steigendem Narkosegasdruck im schliessenden Sinne des Durchflussreglers (40) auf das verschiebbare Teil (90) zur Verschiebung in entgegengesetzte Richtungen einwirken und der Durchflussregler (40) den Durchfluss von Narkosegas durch die 2. Leitung entsprechend der Stellung des verschiebbaren Teiles (90) regelt.

3. Gasverhältnisregelvorrichtung nach Anspruch 2, dadurch gekennzeichnet, dass die 1. Leitung eine 1. Drossel (30) und die 2. Leitung eine 2. Drossel (32) enthalten, die das Verhältnis zwischen dem von den Leitungen in die Sammelleitung (24) zugeführten Narkosegas und Sauerstoff festlegen.

4. Gasverhältnisregelvorrichtung nach Anspruch 3, dadurch gekennzeichnet, dass das verschiebbare Teil (90) beliebig zwischen einer 1. und einer 2. Stellung beweglich ist, wobei in der 1. Stellung der Durchfluss des Narkosegases durch die 2. Leitung einen festgelegten Prozentsatz des Sauerstoffdurchflusses in die Sammelleitung (24) nicht überschreitet.

5. Gasverhältnisregelvorrichtung nach Anspruch 4, dadurch gekennzeichnet, dass bei einer Mittelstellung des verschiebbaren Teiles zwischen der 1. und 2. Stellung der Durchfluss des Narkosegases durch die 2. Leitung gleich dem festgelegten Prozentsatz des Sauerstoffdurchflusses ist.

6. Gasverhältnisregelvorrichtung nach Anspruch 5, dadurch gekennzeichnet, dass der Durchflussregler (40) ein Ventil (94, 102), der Sauerstoff-Druckaufnehmer (42) eine 1. bewegliche Membrangruppe (78), der Narkosegas-Druckaufnehmer (44) eine 2. bewegliche Membrangruppe (78) und das verschiebbare Teil Verbindungsstücke enthalten, die die 1. Membrangruppe (78), die 2. Membrangruppe (78) und das Ventil (94/102) verbinden.

**Claims**

1. Gas ratio regulating device for use in narcosis apparatus, having a first line (48, 64) for supplying oxygen and a second line (105, 68, 72) for supplying narcotic gas to a collecting line (24), each one having an independently adjustable adjusting element (26, 28) for the gas flow in both lines, with an oyxgen pressure receiver (42) at the first line downstream of the adjusting element (26), which emits a first signal corresponding to the oxygen pressure, with a narcotic gas pressure receiver (44) at the second line downstream of the adjusting element (28), which emits a second signal corresponding to the narcotic gas pressure, and with an adjusting device (160, 90, 186, 190) which responds to the pressure difference monitored by both pressure receivers (42, 44), characterised in that it contains a flow regulator (40), connected with the second line (105, 68), for regulating the flow of gas through the second line, which is acted upon by the adjusting device (160, 90, 186, 190).

2. Gas ratio regulating device according to claim 1, characterised in that the oxygen pressure receiver (42) has an output element, the position of which is dependent upon the oxygen pressure downstream of the adjusting element (26) and represents the first signal, and the narcotic gas pressure receiver (44) has an output element, the position of which is dependent upon the narcotic gas pressure downstream of the adjusting element (28) and represents the second signal, and the adjusting device has a displaceable part (90) which is connected with both output elements and with the flow regulator (40), for the purpose of displacement in opposite directions, the output element of the oxygen pressure receiver (42) acting on the displaceable part (90) so as to open the flow regulator (40) when there is a rise in oxygen pressure, and the output element of the narcotic gas pressure receiver (44) acting on the displaceable part (90) so as to close the flow regulator (40) when there is a rise in narcotic gas pressure, and the flow regulator (40) regulating the flow of narcotic gas through the second line in accordance with the position of the displaceable part (90).

3. Gas ratio regulating device according to claim 2, characterised in that the first line contains a first choke (30) and the second line contains a second choke (32), which chokes fix the ratio between the narcotic gas and oxygen supplied by the lines to the collecting line (24).

4. Gas ratio regulating device according to claim 3, characterised in that the displaceable part (90) is movable arbitrarily between a first position and a second position, in the first position the flow of narcotic gas through the second line not exceeding a fixed percentage of the flow of oxygen to the collecting line (24).

5. Gas ratio regulating device according to claim 4, characterised in that when the displaceable part is in mid-position between the first and second position, the flow of narcotic gas through the second line is equal to the fixed percentage of the flow of oxygen.

6. Gas ratio regulating device according to claim 5, characterised in that the flow regulator (40) contains a valve (94/102), the oxygen pressure receiver (42) contains a first movable membrane group (78), the narcotic gas pressure receiver (44) contains a second movable membrane group (78) and the displaceable part contains connecting pieces which connect the first membrane group (78), the second membrane group (78) and the valve (194/102).

**Revendications**

1. Dispositif de régulation du rapport entre des gaz, destiné à être utilisé avec des appareils d'anesthésie comprenant une première conduite (48, 64) destinée à l'introduction de l'oxygène et une seconde conduite (105, 68, 72) destinée à l'introduction de gaz anesthésique dans une conduite collectrice (24), comprenant un organe de réglage (26, 28) réglable indépendamment pour régler le débit de gaz dans chacune des deux conduites, cet organe comprenant un capteur de pression d'oxygène (42) raccordé à la première conduite en aval de l'organe de réglage (26) et qui émet un premier signal en fonction de la pression d'oxygène, un capteur de pression de gaz anesthésique (44) raccordé à la seconde conduite en aval de l'organe de réglage (28), qui émet un second signal en fonction de la pression de gaz anesthésique et un dispositif de commande (160, 90, 186, 190) qui répond à la différence de pression surveillée par les deux capteurs de pression (42, 44), caractérisé en ce qu'il comprend un régulateur de débit (40) relié à la seconde conduite (105, 68) et servant à régler le débit de gaz passant dans la seconde conduite, et sur lequel agit le dispositif de commande (160, 90, 186, 190).

2. Dispositif de régulation du rapport entre des gaz, caractérisé en ce que le capteur de pression d'oxygène (42) comprend un élément de sortie dont la position dépend de la pression d'oxygène en aval de l'organe de réglage (26) et représente le premier signal, en ce que le capteur de pression de gaz anesthésique (44) comprend un élément de sortie dont la position dépend de la pression de gaz anesthésique en aval de l'organe de réglage (28) et représente le second signal, et en ce que le dispositif de commande possède un élément coulissant (90) qui est relié aux deux éléments de sortie et au régulateur de débit (40), l'élément de sortie du capteur de pression d'oxygène (42) et l'élément de sortie du capteur de pression de gaz anesthésique (44) agissant sur l'élément mobile (90) pour le déplacer dans des sens opposés, le premier dans le sens de l'ouverture du régulateur de débit (40) en présence d'un accroissement de la pression d'oxygène et le second dans le sens de la fermeture du régulateur de débit (40) en présence d'un accroissement de la pression de gaz anesthésique , le régulateur de débit (40) réglant le débit de gaz anesthésique qui circule dans la seconde conduite en fonction de la position de l'élément mobile (90).

3. Dispositif de régulation du rapport entre des gaz selon la revendication 2, caractérisé en ce que la première conduite renferme un premier étranglement (30) et la seconde conduite un second étranglement (32), ces étranglements fixant le rapport entre le gaz anesthésique et l'oxygène qui sont acheminés à la conduite collectrice (24) en provenance des conduites.

4. Dispositif de régulation du rapport entre des gaz selon la revendication 3, caractérisé en ce que l'élément mobile (90) peut se déplacer à volonté entre une première position et une seconde position, le débit de gaz anesthésique passant dans la seconde conduite, dans la première position, n'excédant pas un pourcentage fixe du débit d'oxygène envoyé à la conduite collectrice (24).

5. Dispositif de régulation du rapport entre des gaz selon la revendication 4, caractérisé en ce que, dans une position centrale de l'élément mobile qui est comprise entre la première et la seconde position, le débit du gaz anesthésique passant dans la seconde conduite est égal au pourcentage fixé du débit d'oxygène.

6. Dispositif de régulation du rapport entre des gaz selon la revendication 5, caractérisé en ce que le régulateur de débit (40) comprend une soupape (94, 102), en ce que le capteur de pression d'oxygène (42) comprend un premier groupe membrane mobile (78), en ce que le capteur de pression de gaz anesthésique (44) comprend un second groupe membrane mobile (78) et en ce que l'élément mobile comprend des éléments de liaison qui relient le premier groupe membrane (78), le second groupe membrane (78) et la soupape (94/102).

0 039 932

22

FIG.1

9

FIG.2